# EUROPEAN PATENT APPLICATION

(11) **EP 3 093 037 A1**
(43) Date of publication of application: **16.11.2016**
(21) Application number: 15167450.4
(22) Date of filing: 13.05.2015
(51) Int. Cl.: A61M 25/01, A61M 25/09

(54) **GUIDEWIRE**

(71) Applicant: Otto-von-Guericke-Universität Magdeburg, 39106 Magdeburg (DE)
(72) Inventor: Friebe, Michael, 45657 Rechlinghausen (DE); Boese, Axel, 39106 Magdeburg (DE); Krug, Johannes, 39106 Magdeburg (DE)
(74) Representative: Hoyng Rokh Monegier LLP

(57) **Abstract**

The present invention relates to MRI-safe guidewires and to guidewires with multiple elongated shape memory alloy cores 1,2.

## Description

### FIELD OF THE INVENTION

The present invention relates to steerable guidewires and catheters and more particularly to steerable guidewires and catheters that are MRI-safe. Further, the present invention relates to guidewires and catheters having multiple shape memory alloy cores.

### BACKGROUND ART

The purpose of a guidewire is to create a pathway in the vascular system up to the target lesion. The guidewire is like a rail on which the catheter runs. The catheter is used for delivering a medical device to the target lesion. The vascular path is characterized by a severe tortuosity and numerous side branches; this is the reason why guidewires need to be steerable and torquable, in particular in the brain when they need to reach a specific target area to treat, for example, an aneurysm or a stenosis. Other applications of the guidewires are in the diagnosis and treatment of cardiac problems like coronary artery disease, heart valve treatments, peripheral repair work, renal artery stenosis, or even outside the vascular structure by moving through the bronchial.

Current guidewires are usually made of an inner core wire surrounded by an outer coil wrap. The core wire has a tapered shape in the distal portion, which does not extend to the guide wire tip, where it is more flexible. In order to improve lubricity, the guidewire surface is coated with a hydrophilic polymer. The inner core of guidewires is usually made of stainless steel, which provides enhanced torque controllability and pushability. However, stainless steel cores are less flexible and are more susceptible to kinking and deformation.

Recently, unique properties of shape memory alloys (SMA) allowed the development of new generation of guidewires. For example, Nitinol is used to increase the dexterity of the surgeon and to provide more steerablity and flexibility. Nitinol is also used to produce catheter shafts with high deformability and the capability to recover the original shape. However, since Nitinol guidewires possess low rigidity the pushability and torquability of the guidewires are insufficient. Now days, both Nitinol and stainless steel are used for inner core of guidewires.

The system of guidewire and the catheter can be monitored through, for example, real-time X-ray images. Magnetic resonance imaging (MRI) has the potential to offer more advantages over X-ray methods in obtaining high-quality visualization or precisely locating lesions in small vessels. However, MRI-safe and compatible guidewires cannot feature long electrically conductive materials. The current guidewires containing stainless steel and/or Nitinol cores are non-MRI safe, which cause heating and electric current in the MR scanner and potential injuries.

Therefore, it would be desirable to develop a guidewire and a catheter that is compatible with MRI and can solve the above-mentioned problems. Further, it would be desirable to develop a guidewire and a catheter with enhanced torquability, steerablity, and pushability.

To solve the problems mentioned above, the present invention provides such guidewires and catheters that are MRI compatible and have increased torquability, steerablity and pushability. Other features and advantages of the invention will be apparent from the following description and from the claims.

### DISCLOSURE OF THE INVENTION

### SUMMARY OF THE INVENTION

The present invention relates to a guidewire comprising an elongated shape memory alloy core (1) having a distal end and a proximal end, a portion (3) coupled to the proximal end of the elongated shape memory alloy core (1), and a coating, wherein the portion (3) comprising a LC circuit.

The present invention further provides a guide wire comprising a first elongated shape memory alloy core (1) having a distal end and a proximal end, a second elongated shape memory alloy core (2) having a distal end and a proximal end substantially parallel to the shape memory alloy core (1), and a coating. Optionally, the length and/or type of the shape memory alloy cores (1) and (2) are different.

In further embodiment, a portion (3) and/or (4) is coupled to the proximal end of the elongated shape memory alloy core (1) and/or (2) according to the preceding embodiment. Optionally, the portions (3) and/or (4) are composed of a different material from the shape memory alloy core (1) and/or (2). Optionally, the portions (3) and/or (4) comprise LC circuits. Optionally, the portions (3) and/or (4) of the present invention comprise a metal, a plastic, LC circuit, a shape memory alloy or combinations thereof.

In further embodiment, the guidewire according to any of the preceding embodiments, comprising a non-conductive material coupled to the proximal end of the guidewire. Optionally, the non-conductive material is coupled to the proximal end of the portion (3) and/or (4).

In further embodiment, the guidewire according to any of the preceding embodiment, wherein the LC circuit being designed to be powered by high-frequency and/or magnetic fields generated from the MRI system and designed to transfer the energy in form of heat to the elongated shape memory alloy core (1) and/or (2).

In further embodiment, the guidewire according to any of the preceding embodiments, wherein the guidewire being designed to be detectable by x-rays, CT scan, magnetic resonance imaging, or combinations thereto.

In further embodiment, the distal and/or the proximal end of the guidewire according to any of the preceding embodiments is an open end being designed to deliver a therapy device through the cores (1) and/or (2).

Additional embodiments provide systems comprising a guidewire according to any of the preceding embodiments and a catheter. Optionally, the system further comprises a therapy device and MRI system , CT Scan system, or x-ray system.

### DRAWINGS

Those of skill in the art will understand that the drawings, described below, are for illustrative purposes only. The drawings are not intended to limit the scope of the present teachings in any way.
FIG. 1 illustrates the catheter according to the present invention with cores (1) and (2), portions (3) and (4), and the coating (6) at its stretched position. As depicted the core (2) has a shorter length relative to core (1).
FIG. 2 illustrates the catheter according to the present invention and as illustrated in FIG. 1 with cores (1) and (2), portions (3) and (4), and the coating (6) after excitation, thereby causing the cores to bend.

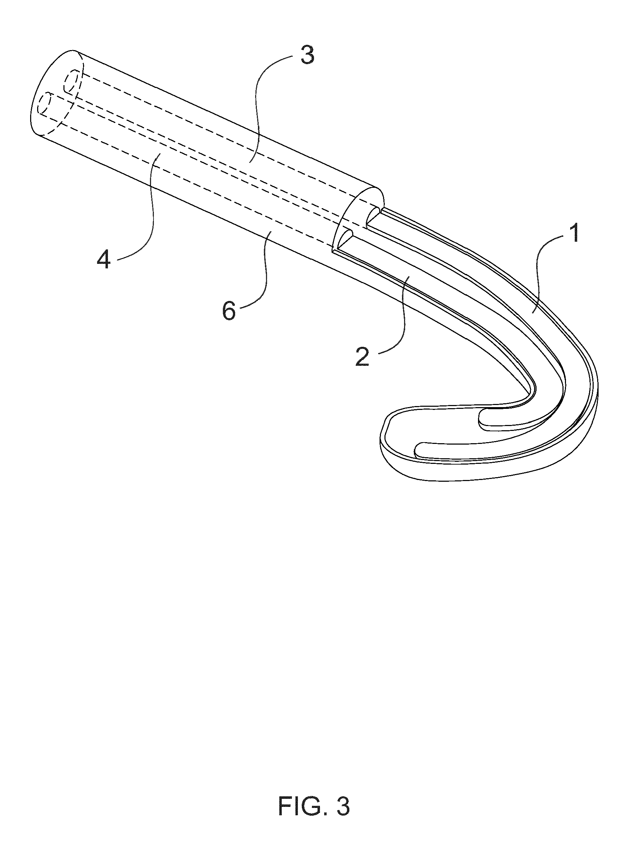

FIG. 3 illustrates the catheter according to the present invention with cores (1) and (2) with different lengths and compositions, portions (3) and (4), and the coating (6) after excitation causing the bend. As depicted, the core (2) bends at a different angle relative to core (1).
FIG. 4a illustrates a single core guidewire with core (1) and portion (3) having coating (6) and (7) wherein (7) is grounded.
FIG. 4b illustrates the circuitry of FIG. 4a.
FIG. 5a illustrates a single core guidewire with core (1) and portion (3) comprising a LC circuit, and coating (6) after excitation, thereby causing the core to bend.
FIG. 5b illustrates a single core guidewire with core (1), portion (3) comprising a LC circuit, and a non-conductive material at the proximal end.
FIG. 5c illustrates the circuitry of FIG. 5b.

### DETAILED DESCRIPTION OF THE INVENTION

In order to better understand the invention, the following definitions are provided:

The term "MRI-safe" as used herein means the guidewires, catheters, and their systems are designed to be present in an MRI operating environment without either damage occurring to the device or the tissue of the patient having said guidewire, catheter, or the system of guidewire and catheter device.

The term "shape memory alloy" (SMA) is as generally known applied to group of materials that demonstrate the ability to change shape, stiffness, position, natural frequency, and other mechanical characteristics in response to temperature, electrical currents, or electromagnetic fields. An article made from such materials can be deformed from an original configuration to an altered configuration. The article is said to have shape memory for the reason that upon application of heat, current or electromagnetic field it can be caused to revert, or attempt to revert, from its original configuration to its altered configuration, for example, from straight to bent configuration.

As used herein the term "shape memory alloy core" refers to the cores made entirely or having at least a portion made of SMA.

The terms "distal" and "proximal" as used herein refer to the relative position of the target lesion and the guidewire. The "distal" end of the guidewire is direction toward the end or at the end of the guidewire or the catheter closest to the target lesion where, for example, a therapy device to be delivered. The "proximal" end of the guidewire is direction toward the end or at the end of the guidewire farthest from the target lesion.

As used herein the term "open end" means that the guidewire or catheter defines an opening that is ordinarily open during typical usage. For example, the distal and/or proximal end of the guidewire according to the present invention, in one embodiment, has an open end. It shall mean that the distal and/or the proximal end of the guidewire is not sealed or closed. Optionally, the term shall mean that if it is sealed or closed, it can be opened and/or re-closed. The guidewire of present invention with an "open end" at its distal end is being designed to deliver a therapy device within its lumen or through the core, for example, through cores (1) or (2) of the guidewire according to the present invention.

As used herein, the terms "sliding", "moving", "motion" and the like have the similar meaning as generally known and are interchangeable.

As used herein the term "subject" refers to any animal, such as a mammal like livestock, pets, and preferably a human. Specific examples of "subject" include, but are not limited, to individuals requiring medical assistance, and in particular, requiring tumor ablation treatment.

As used herein the term "elongate" refer to what is generally known as shaft-like structure. Optionally, the elongate has lumen, i.e. it is hollow. Optionally, the elongate is not hollow. It can be made from any desired size, shape, and diameter.

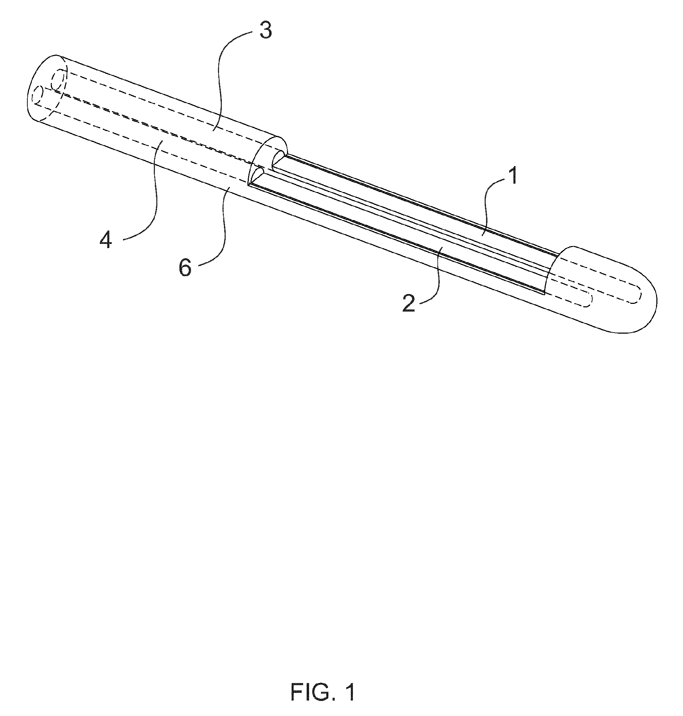

In one aspect of the invention, a guidewire is provided. The guidewire is designed to be detectable by X-ray, CT scan, magnetic resonance, magnetic resonance imaging, or combinations thereof. Referring to the drawings in detail, FIG. 1 illustrates the guidewire of the present invention having two shape memory alloy cores (1) and (2). Optionally, the cores (1) and (2) are the same alloys. They can also have distinct alloys. Optionally, the cores (1) and (2) have the same length or have different lengths. FIG. 1 illustrates that the core (2) is shorter than core (1). The shape memory alloy cores of the present invention can have any length, shape, thickness and diameter. Optionally, the thickness or diameter of the shape memory alloy core (1) and/or (2) is substantially constant along the length of the guidewire.

Further, as depicted by FIG. 1, the guidewire comprises of portions (3) and (4). The portions (3) and (4) can have the same or different composition as cores (1) and/or (2). The portions (3) and/or (4), in one embodiment of the present invention, are non-conductive and in another embodiment of the present invention, are conductive. They can have any length, shape, thickness and diameter. Optionally, the portions (3) and (4) are designed to be MRI-safe. For example, if they are made of conductive materials, their lengths and sized are designed to be MRI-safe. Optionally, the portions (3) and/or (4) comprise a LC circuits. Optionally the portions (3) and/or (4) consist of a LC circuit. Optionally, the portions (3) and (4) comprise a metal, a plastic, LC circuit, a shape memory alloy, or combinations thereof.

In another embodiment, the LC circuits of the present invention are designed to be powered by high frequency and/or magnetic fields generated from MRI system and to transfer the energy in form of heat to the elongated shape memory alloy cores (1) and (2), thereby, causing them to bend.

In another embodiment, the distal and/or proximal end of the guidewire is open (not depicted in the figures) where a therapy device can be moved within the lumen, optionally, beneath the coating (6) of the guidewire, through the cores (1) and/or (2) to be delivered to the target lesion.

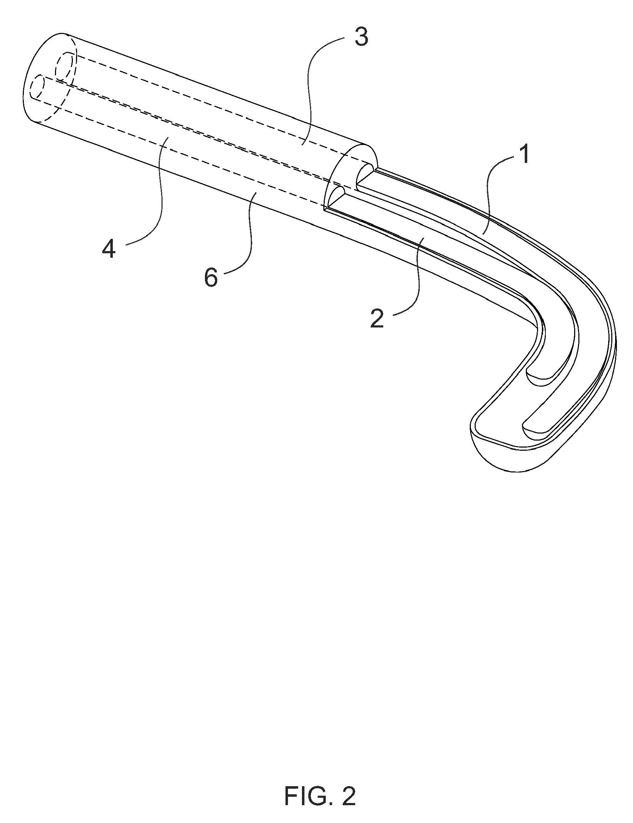

For example, as depicted in FIG. 2, the guidewire is excited through application of a voltage. Excitation can occur, for example, by transferring electrical energy via inductive or capacitive couple using the same or distinct frequencies for cores (1) and (2). Excitation of the guidewire induces change in temperature of the cores (1) and (2), the tip of the guidewire, causing them to bend. The bending angle of the cores (1) and (2) depends, *inter alia,* on the alloy cores and the strengths of the excitation. In normal operating environment (up to 39 degrees Celsius) and without applying electrical energy or excitation, the guidewire is straight. After excitation and subsequent temperature change (40 degrees Celsius or higher) the cores (1) and (2) bend into a specific direction, e.g. 30 degrees away from the axis of the guidewire. This process is reversible or substantially reversible, when the voltage is turned off and the cores cool down again - e.g. through the heat dissipation over the blood flow. Optionally, the catheter or guidewire tip (the distal end of the guidewire) can be a straight metal wire or coiled.

The second core (2) placed parallel (they are parallel before excitation, i.e. before bending occurs) to the first core (1) provides a second additional bend based on the same principle mentioned above with the advantages of increasing the bending angle, for example, 30 degrees to more than 90 degrees which allows the guidewire to enter a branch that is even in the opposite direction of the catheter/guidewires forward motion. Further, it increases the pushability and steerablity of the guidewire.

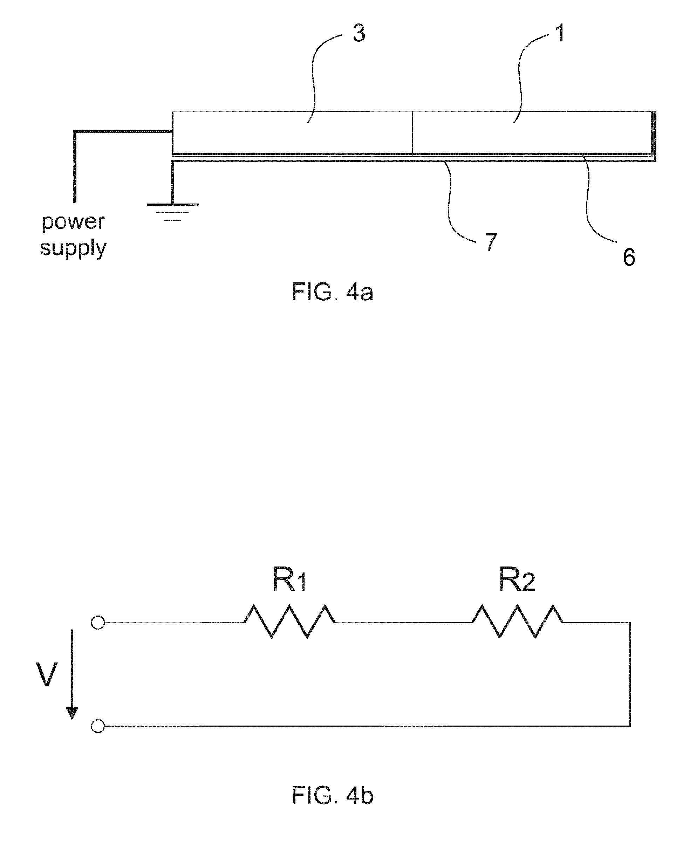

FIG. 4a shows a guidewire according to the present invention with the core (1), the portion (3), the coating (6), and, optionally, another coating layer (7) which is grounded. After application of a voltage, a current flows that causes the core (1) and the portion (3) due to their resistive value (as depicted in FIG. 4b) to heat up. In normal operating environment (up to 39 degrees C) and without voltage applied the catheter/guidewire will be straight. Ohm's law applies here. Through the temperature change (40 degrees or slightly higher) the core (1) bends the tip into a specific direction, e.g. 30 degrees away from the axis of the guidewire. This process is reversible, i.e when the voltage is turned off and the material cools down again - e.g. through the heat dissipation over the blood flow, the catheter tip can return or substantially return to its original configuration. The catheter tip can be a straight metal wire or coiled.

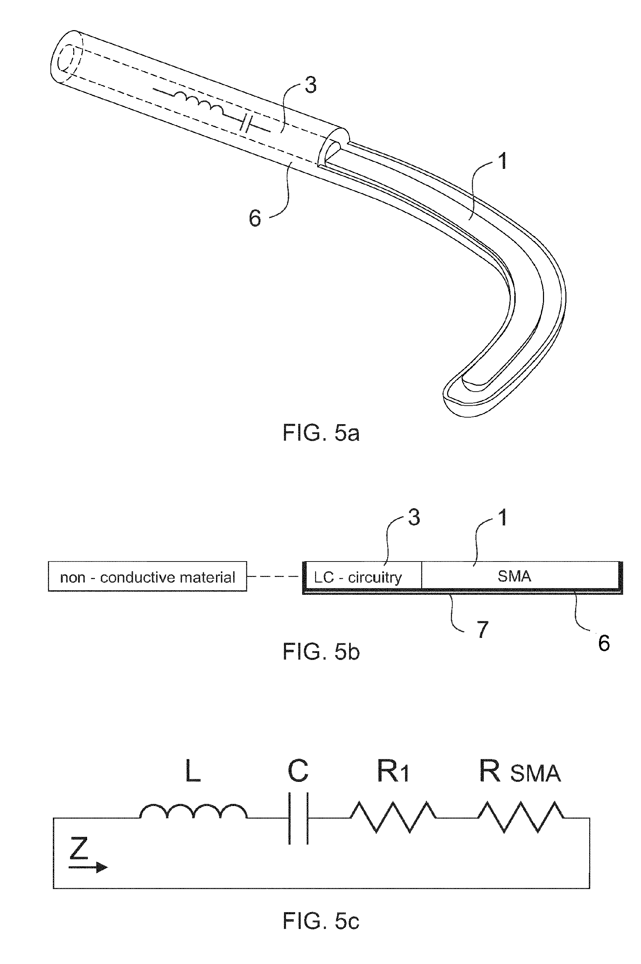

FIG. 5a illustrates the guidewire according to the present invention comprising the core (1), the LC circuit (3), and the coating (6), which operates on the same principle as above. However, in this embodiment, the portion (3) is an LC circuit designed to be powered by high frequency and/or magnetic fields generated from the MRI system and to transfer the energy in form of heat to the cores (1) and (2).

FIG. 5b illustrates another example of the guidewire according to the present invention with the core (1) at the distal end of the guidewire (tip of the guidewire) with, optionally, two to three distinct materials - the material at the tip is a SMA and the second material is a combination of a conductive or non-conductive core with an LC circuit (portion (3) - Optionally, the portion (3) is just an LC circuit; and the remaining material towards the proximal end is made of a nonconductive materials. The LC circuit together with the conductive SMA forms a shortcut resonant circuit with a distinct frequency that depends on the L,C, R values that can be calculated by a skilled person in the art (FIG. 5c). At resonance frequency this circuit receives electrical energy transmitted by an electrical or magnetic field, optionally, through a MRI system, heating the core (1) and subsequently bend as described before. Optionally, a transmitter of electrical energy (e.g. a RF coil) is placed on the guidewire. Optionally, the RF system and the magnetic and RF coils of a MRI system are used to provide this energy. This is achieved through special programs running on the MRI system.

The guidewire of the present invention provides a solution for use with MRI guidance, as it is constructed with a conductor that is short compared to the frequency of the RF field used in a MRI system. Also, the MRI system could be used to provide the energy needed to cause the tip of the core (1) and/or (2) to bend. This form can also be used with X-Ray/CT systems in combination, optionally, with a transmission device for electrical energy or used outside the main magnetic field of a MRI system in combination with such a transmission device.

In further embodiment, the present invention relates to a system comprising the guidewire according to the present invention and a catheter. Optionally, the system further comprises the MRI system, or CT-Scan system or x-ray sytem. Optionally, the system further comprises a therapy device to be delivered to the target lesion.

All embodiments of the present invention as described and depicted on the figures are combinable.

### OTHER EMBODIMENTS

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. The embodiments set-forth above can be performed and combined with other disclosed embodiments according to the invention. For example, the guidewire, the catheter and the system of present invention are combinable. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims.

The phrase "and/or" as used herein in the specification and in the claims, should be understood to mean "either or both".

## Claims

1. A guidewire comprising:
a. a first elongated shape memory alloy core (1) having a distal end and a proximal end,
b. a second elongated shape memory alloy core (2) having a distal end and a proximal end substantially parallel to the shape memory alloy core (1), and
c. a coating.

2. The guidewire according to claim 1, wherein the elongated shape memory alloy core (1) and/or (2) is coupled to a portion (3) and/or (4), respectively.

3. The guidewire according claim 2, wherein the portions (3) and (4) are composed of a different material from the shape memory alloy core (1) and/or (2).

4. The guidewire according claim 2, wherein the portions (3) and/or (4) comprising LC circuit.

5. The guidewire according to claim 2, wherein the portions (3) and/or (4) comprising a metal, a plastic, LC circuit, a shape memory alloy or combinations thereof.

6. The guidewire according to claim 2, wherein the length and/or the material of shape memory alloy cores (1) and (2) are different.

7. The guidewire according to claim 2, wherein the thickness or diameter of the shape memory alloy core (1) and/or (2) is substantially constant along the length of the guide wire.

8. The guidewire according to any of claims 2-7, wherein a non-conductive material is coupled to the proximal end of the portion (3) and/or (4).

9. The guidewire according to claim 4 or 5, wherein the LC circuit being designed to be powered by MRI system and to transfer heat to the elongated shape memory alloy core (1) and/or (2).

10. The guidewire according to any of claims 1-9, wherein the guidewire is designed to be detectable by x-rays, CT-Scan, Magnetic Resonance Imaging and combinations thereto.

11. The guidewire according to any of claims 1-10, wherein the distal end of the guidewire is an open end being designed to deliver a therapy device through the core (1) and/or (2).

12. A guidewire comprising:
a. an elongated shape memory alloy core (1) having a distal end and a proximal end,
b. a portion (3) coupled to the proximal end of the elongated shape memory alloy core (1), wherein the portion (3) comprising a LC circuit, and
c. a coating.

13. The guidewire according to claim 12, wherein the distal end of the guidewire is an open end being designed to deliver a therapy device through the core (1) and/or (2).

14. A system comprising the guidewire according to any of claims 1-13 and a catheter.

15. The system of claim 14, further comprising a MRI system.
